(19) Europäisches Patentamt
European Patent Office
Office européen des brevets

(11) Veröffentlichungsnummer: **0 283 660**
**A2**

# (12) EUROPÄISCHE PATENTANMELDUNG

(21) Anmeldenummer: **88101012.8**

(22) Anmeldetag: **25.01.88**

(51) Int. Cl.⁴: **C07C 45/48**, C07C 49/04, C07C 49/10

(30) Priorität: **25.03.87 DE 3709765**

(43) Veröffentlichungstag der Anmeldung: **28.09.88 Patentblatt 88/39**

(84) Benannte Vertragsstaaten: **BE CH DE FR GB IT LI NL**

(71) Anmelder: **CHEMISCHE WERKE HÜLS AG - RSP Patente / PB 15 - Postfach 13 20 D-4370 Marl 1(DE)**

(72) Erfinder: **Kleine-Homann, Walter, Dr. Buchenallee 14 D-4408 Dülmen(DE)**

## (54) Verfahren zur Herstellung unsymmetrischer aliphatischer Ketone.

(57) Unsymmetrische Ketone werden bisher durch Umsetzung von Aldehyden, von sekundären Alkoholen oder durch Umsetzung von Carbonsäuregemischen in Gegenwart von Polyphosphorsäure - oder radioaktiven Thoriumoxid-Katalysatoren hergestellt. Man benötigte entweder aufwendige und teure Einsatzstoffe oder für den technischen Einsatz ungeeignete Katalysatoren.

Durch Verwendung eines Ceroxid enthaltenden Katalysators auf einem Aluminiumoxid-Träger erhält man aus Carbonsäuregemischen bei hohen Umsätzen und einer guten Selektivität die unsymmetrischen Ketone.

Herstellung der unsymmetrischen Ketone, beispielsweise von Methyl-ethyl-keton, Ethyl-butyl-keton, Methyl-isopropyl-keton oder von Methyl-octyl-keton

EP 0 283 660 A2

## Verfahren zur Herstellung unsymmetrischer aliphatischer Ketone

Die Erfindung betrifft ein Verfahren zur Herstellung unsymmetrischer aliphatischer Ketone, das durch Umsetzung einer Mischung zweier aliphatischer Carbonsäuren bei erhöhten Temperaturen an einem oxidischen Metallkatalysator zu einem Ketongemisch führt, bei dem der Anteil des gewünschten unsymmetrischen Ketons überwiegt.

$$R\text{-}COOH + R'\text{-}COOH \xrightarrow{Kat} R\text{-}\overset{\overset{O}{\|}}{C}\text{-}R + R'\text{-}\overset{\overset{O}{\|}}{C}\text{-}R' + R\text{-}\overset{\overset{O}{\|}}{C}\text{-}R' \quad (I)$$

In der Literatur sind zahlreiche Synthesen von (1) beschrieben wie beispielsweise in der GB-PS 1 194 057. Als Einsatzprodukt dient hier zusätzlich zur Säure ein Aldehyd, wobei die Reaktion in Gegenwart von Wasser abläuft. In der FR-PS 1 533 651 setzt man ebenfalls die teuren Aldehyde als Einsatzstoffe ein. Ein anderes Produktionsverfahren wird in Tetrahedran Lett. 1972, 257 bis 260, beschrieben. Danach werden geeignete sekundäre Alkohole zu den entsprechenden Ketonen dehydriert. Problematisch ist hier die Bereitstellung des erforderlichen sekundären Alkohols.

Auch der Einsatz eines Carbonsäuregemisches zur Herstellung von Ketonen ist beschrieben. Während bei der US-PS 3 468 956 Polyphosphorsäure im Temperaturbereich von 100 bis 300 °C als Katalysator eingesetzt wird, benutzte eine Vielzahl von Autoren radioaktive Thoriumoxidkatalysatoren.

Die bekannten Verfahren benötigen demnach entweder z. T. aufwendige und damit teure Einsatzprodukte oder für den technischen Einsatz ungeeignete Katalysatorsysteme.

Es bestand daher ein Interesse an einem Verfahren, das unter Einsatz leicht verfügbarer Einsatzstoffe und Katalysatorsysteme die Herstellung unsymmetrischer aliphatischer Ketone mit befriedigender Ausbeute ermöglicht.

Die sich hieraus ergebende Aufgabe wird erfindungsgemäß entsprechend den Angaben der Patentansprüche gelöst.

Überraschenderweise ist die bevorzugte Bildung unsymmetrischer aliphatischer Ketone durch den gezielten Einsatz zweier aliphatischer Carbonsäuren unter Verwendung eines Ceroxids enthaltenden Katalysators möglich, wobei man einen Umsatz der Säuremischung von 98 bis 100 % erreicht. Die Selektivität der Bildung des unsymmetrischen Ketons, auf die höhermolekulare Carbonsäure bezogen, liegt im allgemeinen bei 40 bis 60 %. Nach Fraktionierung des Rohproduktes, beispielsweise in einer Multifilkolonne, erhält man die unsymmetrischen Ketone in hoher Reinheit, z. B. von 99,6 %.

Bei dem Katalysator handelt es sich um einen Aluminiumoxid-Träger, der mit Ceroxid, vorzugsweise mit 5 bis 25 %, insbesondere mit 8 bis 15 %, Ceroxid beladen ist. Während bei niedrigeren Ceroxid-Konzentrationen bei vergleichbaren Reaktionstemperaturen eine Umsatzreduzierung festzustellen ist, ergeben höhere Konzentrationen keine nennenswerte Aktivitätssteigerung. Die Reaktion erfolgt bei Temperaturen von 320 bis 550 °C, bevorzugt bei 380 bis 450 °C. Bei höheren Temperaturen ist ein merklicher Selektivitätsverlust festzustellen, während bei niedrigeren kein wirtschaftlicher Umsatz mehr erreicht wird.

Als Einsatzstoffe eignen sich Alkylcarbonsäuren, die geradkettig wie auch verzweigt sein können. Die Mischung der eingesetzten Säuren erfolgt zweckmäßigerweise vor der Dosierung. Selbstverständlich ist auch eine gleichzeitige aber lokal getrennte Zudosierung des jeweiligen Säurepaars möglich.

Das gewünschte Mengenverhältnis ist dabei jedoch ständig zu überprüfen und sicherzustellen, da andernfalls deutliche Ausbeuteverluste auftreten.

Als Mischungsverhältnis der Carbonsäuren hat sich ein Molverhältnis von 10 bis 1 : 1, bevorzugt von 4 bis 2 : 1, bewährt, wobei die Komponente mit dem geringeren Molekulargewicht im Regelfall überwiegt.

Die erhaltenen unsymmetrischen Ketone verwendet man als Speziallösemittel, Riechstoffkomponenten oder Riechstoffvorprodukte.

### Beispiele

200 ml des Kontaktes werden in einem beheizbaren Quarzrohr von 0,5 m Länge und 26 mm Durchmesser vorgelegt. Unter Zugabe von Stickstoff wird die Füllung bis zur erwünschten Temperatur (siehe Tabelle) erwärmt. Die Temperaturerfassung erfolgt mit Hilfe eines Widerstandsthermometers, das sich in einem zentrierten Quarzrohr von 6 mm Durchmesser befindet und dort je nach Anforderung in die

gewünschte axiale Position verschoben werden kann. Das vorbereitete Carbonsäuregemisch wird sodann über einen Verdampfer mit nachgeschaltetem Überhitzer auf die gewünschte Reaktionstemperatur erhitzt und über den temperierten Katalysator gefahren. Der Reaktoraustrag wird kondensiert und gaschromatographisch analysiert. Die Bestätigung der Werte erfolgt im Regelfall durch Fraktionierung des Rohproduktes an einer 0,5 m Multifilkolonne. Durch Regelung der Reaktionstemperatur wird ein Umsatz der Säuremischung von 98 bis 100 % eingestellt.

| Beispiel | Säuregemisch | Mischungsanteil Molverhältnis | Katalysator | Reaktionstemp. (° C) | Produkt | Selektivität* (%) | Reinheit (%) |
|---|---|---|---|---|---|---|---|
| 1 | Essigsäure<br>Propionsäure | 1<br>1 | A | 450 | Methyl-<br>ethyl-keton | 42 | 99,0 |
| 2 | Essigsäure<br>Propionsäure | 2<br>1 | A | 400 | Methyl-<br>ethyl-keton | 51 | 98,8 |
| 3 | Essigsäure<br>Propionsäure | 4<br>1 | A | 420 | Methyl-<br>ethyl-keton | 48 | 99,2 |
| 4 | Essigsäure<br>Propionsäure | 1<br>2 | A | 420 | Methyl-<br>ethyl-keton | 35 | 99,0 |
| 5 | Propionsäure<br>Valeriansäure | 2<br>1 | B | 430 | Ethyl-<br>butyl-keton | 36 | 98,5 |
| 6 | Propionsäure<br>Valeriansäure | 2<br>1 | C | 440 | Ethyl-<br>butyl-keton | 50 | 98,5 |
| 7 | Propionsäure<br>Valeriansäure | 2<br>1 | D | 420 | Ethyl-<br>butyl-keton | 46 | 98,8 |
| 8 | Essigsäure<br>Isobuttersäure | 2<br>1 | A | 420 | Methyl-iso-<br>propyl-keton | 56 | 99,6 |
| 9 | Essigsäure<br>Pelargonsäure | 3<br>1 | C | 420 | Methyl-<br>octyl-keton | 57 | 98,6 |

Katalysator A 12 % Ceroxid auf Aluminiumoxid B 1 % Ceroxid auf Aluminiumoxid

C 50 Teile Katalysator D + 50 Teile Aluminiumoxid D 10 % Ceroxid auf Aluminiumoxid

* Die Selektivität der Bildung des unsymmetrischen Ketons ist auf die höhermolekulare Carbonsäure bezogen.

## Ansprüche

1. Verfahren zur Herstellung unsymmetrischer Ketone durch Umsetzung von zwei aliphatischen Carbonsäuren bei erhöhten Temperaturen an einem oxidischen Metallkatalysator,
dadurch gekennzeichnet,
daß man die Umsetzung der zwei aliphatischen Carbonsäuren in einem Molverhältnis von 10 bis 1 : 1 bei Temperaturen von 320 bis 550 °C an einem Ceroxid enthaltenden Katalysator auf einem Aluminiumoxid-Träger durchführt und das erhaltene Rohprodukt in bekannter Weise fraktioniert.

2. Verfahren nach Anspruch 1,
dadurch gekennzeichnet,
daß man einen Ceroxid-Katalysator einsetzt, der 5 bis 25 % Ceroxid auf einem Aluminiumoxid-Träger enthält.

3. Verfahren nach Anspruch 1 und 2,
dadurch gekennzeichnet,
daß man einen Ceroxid-Katalysator einsetzt, der 8 bis 15 % Ceroxid auf einem Aluminiumoxid-Träger enthält.

4. Verfahren nach einem der Ansprüche 1 bis 3,
dadurch gekennzeichnet,
daß man die Umsetzung bei einem Molverhältnis der zwei aliphatischen Carbonsäuren von 4 bis 2 : 1 durchführt.

5. Verfahren nach einem der Ansprüche 1 bis 4,
dadurch gekennzeichnet,
daß man die Umsetzung bei Temperaturen von 380 bis 450 °C durchführt.

6. Verfahren nach einem der Ansprüche 1 bis 5,
dadurch gekennzeichnet,
daß man die beiden aliphatischen Carbonsäuren vor der Zugabe mischt.